# EUROPEAN PATENT SPECIFICATION

(11) **EP 1 511 445 B1**
(45) Date of publication and mention of the grant of the patent: **05.05.2010**
(21) Application number: 03753088.8
(22) Date of filing: 16.05.2003
(51) Int. Cl.: A61F 2/02, A61F 2/24, A61F 2/28, A61L 2/00, A61L 2/08, A61L 2/20

(54) **STERILIZED XENOGRAFT TISSUE**
STERILISIERTES XENOTRANSPLANTATGEWEBE
XENOGREFFE TISSULAIRE STERILISEE

(30) Priority: 17.05.2002 US 150670
(43) Date of publication of application: 09.03.2005
(73) Proprietor: Aperion Biologics, Inc., San Antonio, TX 78247 (US)
(72) Inventor: STONE, Kevin, R., Mill Valley, CA 94941 (US); TUREK, Thomas, J., San Francisco, CA 94132 (US)
(74) Representative: Hill, Justin John
(86) International application number: PCT/US2003/015630
(87) International publication number: WO 2003/097809

(56) References cited:
- WO-A-99/47080
- WO-A-02/089711
- US-A- 4 361 586
- US-A- 5 263 984
- US-A1- 2001 039 459
- US-A1- 2003 068 815
- US-B1- 6 231 608
- DZIEDZIC-GOCLAWSKA ET AL: 'Sterilization of tissue allografts' ADVANCES IN TISSUE BANKING vol. 1, 1996, pages 261 - 321, XP002971643

## Description

### FIELD OF THE INVENTION

The present invention relates to the field of treatment of defective human tissue, and in particular, to replacement and repair of defective or damaged human tissue using a substantially immunologically compatible xenograft material from a non-human animal.

### BACKGROUND OF THE INVENTION

Xenotransplantation is a procedure that involves the transplantation, implantation, or infusion into a human recipient of either (a) live cells, tissues, or organs from a nonhuman animal source or (b. ) human body fluids, cells, tissues or organs that have had ex vivo contact with live nonhuman animal cells, tissues, or organs. Tissue for allograft transplantation is commonly cryopreserved to optimize cell viability during storage, as disclosed, for example, in U. S. Pat. Nos. 5,071, 741; 5,131, 850 ; 5,160, 313 and No. 5,17I, 660.

Once implanted in an individual, a xenograft can provoke immunogenic reactions such as chronic and hyperacute rejection of the xenograft. Xenograft materials may be chemically treated to reduce immunogenicity prior to implantation into a recipient. For example, glutaraldehyde is used to cross-link or "tan" xenograft tissue in order to reduce its antigenicity, as described in detail in U. S. Pat. No. 4,755, 593. Other agents such as aliphatic and aromatic diamine compounds may provide additional crosslinking through the side chain carboxyl groups of aspartic and glutamic acid residues of the collagen polypeptide. Glutaraldehyde and diamine tanning also increases the stability of the xenograft tissue. However, there remains a need in the xenotransplantation art for a substantially non-immunogenic xenograft material.

In addition, although American Association of Tissue Banks standards (Woll JE et al., Standards for Tissue Banking. (American Association of Tissue Banks, McLean, Virginia, 2001)) recommend that cultures be obtained before and after processing, these standards do not address the potential problem of baeteriostasis after processing or specify a culture method. MMWR 51(10); 207-210 (March 15, 2002). Aseptic processing does not eradicate contamination with organisms (CDC, "Septic arthritis following anterior cruciate ligament reconstruction using tendon allografts---Florida and Louisiana, 2000." MMWR 50: 1081-3 (2001)), and antibiotic/antifungal solutions will not eliminate spores of organisms such as chlostridial bacteria. Accordingly, there is a need in the xenotransplantation art for an effective method of sterilizing the substantially non-immunogenic xenograft material. International Patent Publication No. 02/089711 discloses a substantially non-immunogenic submucosal xenograft for implantation into humans.

EP 1392202, which is comprised in the state of the art under Art.54(3) EPC, discloses methods for processing submucosal xenografts, including cellular disruption treatment and glycosidase digestion of carbohydrate moieties of the xenograft; treatment with radiation, in particular gamma radiation; one or more cycles of freezing and drawing; treatment with a chemical cross-linking agent; and treatment with alcohol or ozonation.

US 6231608 discloses methods for producing bone or soft tissue xenografts, including treatment with radiation (in particular gamma radiation), one or more cycles of freezing and thawing, treatment with a chemical cross-linking agent; treatment with alcohol or ozonation; cellular disruption treatment; glycosidase digestion of carbohydrate moieties of the xenograft, or treatment with proteoglycan-depleting factors.

### SUMMARY OF THE INVENTION

The invention provides a method of sterilizing a substantially non-immunogenic xenograft material for implantation into a human. The invention provides a method of sterilizing xenograft material for implantation into a human, comprising the steps of (1) obtaining substantially non-immunogenic xenograft material; (2) treating the xenograft material with at least one crosslinking agent; and (3) subjecting the crosslinked xenograft material to radiation treatment. In addition the methods of the invention include, in any order, a cellular disruption treatment, glycosidase digestion of carbohydrate moieties of the xenograft, or treatment with proteoglycan-depleting factors. Optionally, the xenograft can be exposed to an aldehyde for further crosslinking. After one or more of the above-described processing steps, the methods of the invention provide a xenograft having substantially the same mechanical properties as the corresponding native human tissue.

In one embodiment, the method of the invention is the additive combination of chemical and terminal treatments. This embodiment advantageously provides a dose validation for product irradiation sterilization, applicable to electron beam irradiation.

In a particular embodiment, electron beam level is dictated by ANSI/AAMI/ISO 11137 sterility assurance limits (10-6) through sub-lethal dose assessment. This yields a validated dose, in our case for our process of 17.8 kGy or 1.78 mRads (interchangeable, with the former terminology used industrially).

The 17.8 kGy dose is consistent with publications by those skilled in the art of investigating radiation and graft integrity. Glutaraldehyde cross-linking is used to stabilize the collagen structure, attenuate immunological recognition of the graft, and is additive in sterilization effect with respect to viral inactivation. As shown by the supporting biomechanical data (*see*, EXAMPLE 3), this embodiment provides a post-processing device of the invention having advantageous integrity and implantability.

In another embodiment, the invention provides a method of preparing a xenograft for implantation into a human, which includes removing at least a portion of soft tissue from a non-human animal to provide a xenograft; washing the xenograft in water and alcohol; and subjecting the xenograft to at least one treatment selected from the group consisting of exposure to ultraviolet radiation, immersion in alcohol, ozonation, and freeze/thaw cycling, whereby the xenograft has substantially the same mechanical properties as the corresponding native human tissue.

In a further embodiment, the invention provides a sterilized, substantially nonimmunogenic xenograft for implantation into a human, wherein the xenograft has substantially no surface carbohydrate moieties which are susceptible to glycosidase digestion, and whereby the portion has substantially the same mechanical properties as the corresponding native human tissue.

### BRIEF DESCRIPTION OF THE DRAWINGS

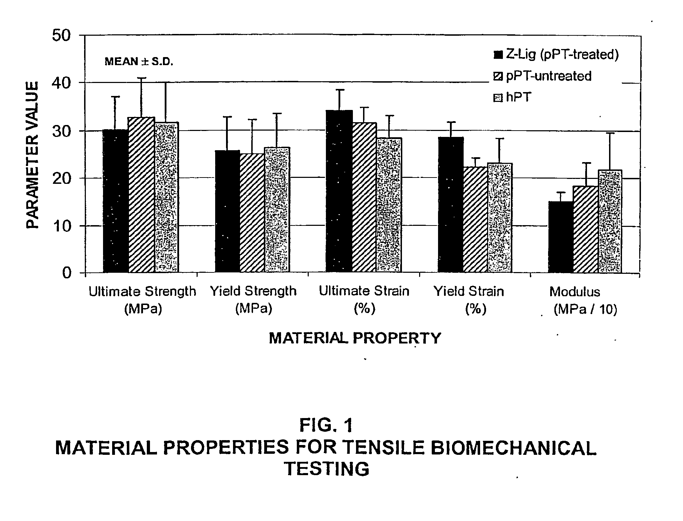

FIG. 1 is a graphical comparison of group material properties, with the modulus reported in MPa x 10⁻¹ for comparison purposes. No significant differences were found between treated and untreated porcine test groups in the parameters of ultimate strength, yield strength or ultimate strain. Small, non-significant differences did exist in yield strain and modulus between treated and untreated porcine groups. These differences are attributed to tissue hydration due to processing and storage and do not represent meaningful differences in material properties before and after treatment. The human allograft group is presented for comparative purposes. Significant differences were not found between porcine groups and the human grafts in the parameters of ultimate strength, yield strength or ultimate strain or yield strain. Modulus differences did exist between the porcine and human groups, but this difference is well within acceptable values for ACL reconstruction grafts and probably attributed to the compliant nature of ligaments harvested from young (6 to 10 month old) swine as compared to mature human donors:

### DETAILED DESCRIPTION OF THE INVENTION

*Definitions.* As used herein, the term "xenograft" is synonymous with the term "heterograft" and refers to a graft transferred from an animal of one species to one of another species. Stedman's Medical Dictionary (Williams & Wilkins, Baltimore, MD, 1995). As used herein, the term "xenogeneic", refers to tissue transferred from an animal of one species to one of another species. *Id.* Replacement of articular cartilage can also be by allografting (Transplants made from one person or animal to another in the same species ("allogeneic"); Sengupta et al. (1974) J. Bone Suro. 56B(1):167-177; Rodrigo et al. (1978) Clin Orth. 134:342-349). With respect to soft tissue for xenografts, porcine peritoneum or pericardium can be harvested to form allografts or xenografts according to procedures known to those of ordinary skill in the art. *See, for example,* the peritoneum harvesting procedure discussed in U.S. Patent No. 4,755,593.

As used herein, the term "cellular disruption" as in, for example, cellular disruption treatment, refers to a treatment for killing cells. Xenograft tissues may also be subjected to various physical treatments in preparation for implantation. For example, U.S. Pat. No. 4,755,593 discloses subjecting xenograft tissue to mechanical strain by stretching to produce a thinner and stiffer biomaterial for grafting. Tissue for allograft transplantation is commonly cryopreserved to optimize cell viability during storage, as disclosed, for example, in U.S. Pat. No. 5,071,741; U.S. Pat. No. 5,131,850; U.S. Pat. No. 5,160,313; and U.S. Pat. No. 5,171,660. U.S. Pat. No. 5,071,741 discloses that freezing tissues causes mechanical injuries to cells therein because of extracellular or intracellular ice crystal formation and osmotic dehydration. The term "extracellular components", as used herein, refers to any extracellular water, collagen and elastic fibers, proteoglycans, fibronectin, elastin, and other glycoproteins, such as are present in vertebrate tissue.

The term "soft tissue", as used herein, refers to cartilaginous structures, such as meniscus and articular cartilage; ligaments, such as anterior cruciate ligaments; tendons; and heart valves. Moreover, the femoral condyles articulate with the surface plateaus of the tibia, through the cartilaginous medial and lateral menisci soft tissue, and all of these structures are held in place by various ligaments. The medial and lateral menisci are structures comprised of cells called fibrochondrocytes and an extracellular matrix of collagen and elastic fibers as well as a variety of proteoglycans. Undamaged menisci provide shock absorption for the knee by ensuring proper force distribution, stabilization, and lubrication for the interacting bone surfaces within the knee joint, which are routinely exposed to repeated compression loading during normal activity. Much of the shock absorbing function of the medial and lateral menisci is derived from the elastic properties inherent to cartilage. When menisci are damaged through injury, disease, or inflammation, arthritic changes occur in the knee joint, with consequent loss of function.

As used herein, the term "portion" refers to all or less than all of the respective soft tissue xenograft material.

The term "chronic rejection", as used herein, refers to an immunological reaction in an individual against a xenograft being implanted into the individual. Typically, chronic rejection is mediated by the interaction of IgG natural antibodies in the serum of the individual receiving the xenograft and carbohydrate moieties expressed on cells, and/or cellular matrices and/or extracellular components of the xenograft. For example, transplantation of xenografts from nonprimate mammals (*e*.*g*., porcine or bovine origin) into humans is primarily prevented by the interaction between the IgG natural anti-Gal antibody present in the serum of humans with the carbohydrate structure Galα1-3Ga1β1-4G1cNAc-R (α-galactosyl or α-gal epitope) expressed in the xenograft. Stone KR et al., "Porcine and bovine cartilage transplants in cynomolgus monkey: 1. A model for chronic xenograft rejection." Transplantation 63: 640-645 (1997); Galili U. et al., "Porcine and bovine cartilage transplants in cynomolgus monkey: II. Changes in anti-Gal response during chronic rejection." Transplantation 63: 646-651 (1997). In chronic rejection, the immune system typically responds within one to two weeks of implantation of the xenograft.

In contrast with "chronic rejection", the term "hyperacute rejection" as used herein, refers to the immunological reaction in an individual against a xenograft being implanted into the individual, where the rejection is typically mediated by the interaction of IgM natural antibodies in the serum of the individual receiving the xenograft and carbohydrate moieties expressed on cells. This interaction activates the complement system, causing lysis of the vascular bed and stoppage of blood flow in the receiving individual within minutes to two to three hours.

As used herein, the term "surface carbohydrate moiety (moieties)" or "first surface carbohydrate moiety (moieties)" refers to a terminal α-galactosyl sugar at the non-reducing end of a carbohydrate chain. As used herein, the term "second surface carbohydrate moiety (moieties)" refers to a *N*-acetyllactosamine residue at the non-reducing end of a carbohydrate chain, the residue being non-capped either naturally or as a result of prior cleavage of an α-galactosyl epitope.

*Tissue Processing*: The invention is directed against the chronic rejection of xenografts for implantation into humans. Accordingly, xenografts produced in accordance with the methods of the invention are substantially non-immunogenic, while generally maintaining the mechanical properties of a corresponding native human tissue.

While the xenograft may undergo some shrinkage during processing, a xenograft prepared in accordance with the invention will have the general appearance of a corresponding native human tissue.

The invention provides, in one embodiment, a method for preparing or processing a xenogeneic tissue for engraftment into humans. The tissue may be harvested from any non-human animal to prepare the xenografts of the invention. Tissue from transgenic non-human animals or from genetically altered non-human animals may also be used as xenografts in accordance with the invention. Preferably, bovine, ovine, or porcine soft tissue, and more preferably porcine soft tissue, serve as sources of soft tissue used to prepare the xenografts. Alternatively, porcine pericardium can be used to form the xenografts of the invention.

In the first step of the method of the invention, an intact soft tissue is removed from a non-human animal. Pericardium may be also harvested and implanted to replace or repair damaged soft tissue by those of skill in the art using known techniques.

In accordance with the invention, the soft tissue is collected from freshly killed animals and preferably immediately placed in a suitable sterile isotonic or other tissue preserving solution. Preferably, harvesting occurs as soon as possible after slaughter of the animal and preferably is performed in the cold, i. e., in the approximate range of about 5°C to about 20°C, to minimize enzymatic degradation of the tissue, under strict sterile technique.

The harvested tissue are dissected free of adjoining tissue. Once removed, optionally, the tissue portions are supported with stents, rings and the like. The portion is carefully identified and dissected free of adhering tissue, plaques, calcifications and the like, thereby forming the xenograft.

In one form of the invention, porcine peritoneum or pericardium is harvested to form xenografts according to procedures known to those of ordinary skill in the art. See, for example, the peritoneum harvesting procedure discussed in U. S. Pat. No. 4,755, 593.

In a preferred form of the invention, the xenograft is then washed in about ten volumes of sterile cold water to remove residual blood proteins and water soluble materials. The xenograft is then immersed in alcohol at room temperature for about five minutes, to sterilize the tissue and to remove non-collagenous materials. After alcohol immersion, the xenograft may be directly implanted or may be subjected to at least one of the following treatments: radiation treatment, treatment with alcohol, ozonation, one or more cycles of freezing and thawing, and/or treatment with a chemical cross-linking agent. When more than one of these treatments is applied to the xenograft, the treatments may occur in any order.

In another embodiment, the xenograft is treated by again being placed in an alcohol solution. Any alcohol solution may be used to perform this treatment. Preferably, the xenograft is placed in a 70% solution of isopropanol at room temperature.

In still another embodiment, the xenograft is subjected to ozonation.

In a further embodiment of the method of the invention, the xenograft is treated by freeze/thaw cycling. For example, the xenograft may be frozen using any method of freezing, so long as the xenograft is completely frozen, *i*.*e*., no interior warm spots remain which contain unfrozen tissue. Preferably, the xenograft is dipped into liquid nitrogen for about five minutes to perform this step of the method. More preferably, the xenograft is frozen slowly by placing it in a freezer. In the next step of the freeze/thaw cycling treatment, the xenograft is thawed by immersion in an isotonic saline bath at room temperature (about 25°C) for about ten minutes. No external heat or radiation source is used, in order to minimize fiber degradation.

In yet a further embodiment, the xenograft optionally is exposed to a chemical agent to tan or crosslink the proteins within the extracellular components, to further diminish or reduce the immunogenic determinants present in the xenograft. Any tanning or crosslinking agent may be used for this treatment, and more than one crosslinking step may be performed or more than one crosslinking agent may be used in order to ensure complete crosslinking and thus optimally reduce the immunogenicity of the xenograft. For example, aldehydes such as glutaraldehyde, formaldehyde, adipic dialdehyde, and the like, may be used to crosslink the extracellular collagen of the xenograft in accordance with the method of the invention. Other suitable crosslinking agents include aliphatic and aromatic diamines, carbodiimides, diisocyanates, and the like.

When an aldehyde such as, for example, glutaraldehyde is used as the crosslinking agent, the xenograft may be placed in a buffered solution containing about 0.001% to about 5.0% glutaraldehyde and preferably, about 0.01 % to about 5.0% glutaraldehyde, and having a pH of about 7.4. More preferably about (0.01 % to about 1.0%) aldehyde, and most preferably about (0.01 % to about 0.20%) aldehyde is used. Any suitable buffer may be used, such as phosphate buffered saline or trishydroxymethylaminomethane, and the like, so long as it is possible to maintain control over the pH of the solution for the duration of the crosslinking reaction, which may be from one to fourteen days, and preferably from one to five days, and most preferably from three to five days.

Alternatively, the xenograft can be exposed to a crosslinking agent in a vapor form, including, but not limited to, a vaporized aldehyde crosslinking agent, such as, for example, vaporized formaldehyde. The vaporized crosslinking agent can have a concentration and a pH and the xenograft can be exposed to the vaporized crosslinking agent for a period of time suitable to permit the crosslink-ing reaction to occur. For example, the xenograft can be exposed to vaporized crosslinking agent having a concentration of about 0.001% to about 5.0% and preferably, about 0.01% to about 5.0%, and a pH of about 7.4. More preferably, the xenograft is exposed to the aldehyde in an amount ranging from about 0.01 % to about 0.10%, and most preferably to an aldehyde ranging in an amount from about 0.01% to about 0.05%. The xenograft is exposed to the aldehyde for a period of time, which can be from one to fourteen days, and preferably from one to five days, and most preferably from three to five days. Exposure to vaporized crosslinking agent can result in reduced residual chemicals in the xenograft from the crosslinking agent exposure.

The crosslinking reaction continues until the immunogenic determinants are substantially eliminated from the xenogeneic tissue, but the reaction is terminated prior to significant alterations of the mechanical properties of the xenograft. When diamines are also used as crosslinking agents, the glutaraldehyde crosslinking occurs after the diamine crosslinking, so that any unreacted diamines are capped. After the crosslinking reactions have proceeded to completion as described above, the xenograft is rinsed to remove residual chemicals, and (0.01-0.50 M) glycine, and preferably, (0.01-0.20 M) glycine is added to cap any unreacted aldehyde groups which remain.

In addition to the above treatments, the xenograft is subjected to a cellular disruption treatment to kill the xenograft's cells. The cellular disruption treatment precedes or follows digestion of the xenograft with glycosidases to remove surface carbohydrate moieties from the xenograft. In addition or in lieu of the glycosidase treatment, either preceding or following the glycosidase treatment, the xenograft may be treated with proteoglycan-depleting factors.

The xenograft is subjected to a cellular disruption treatment to kill the cells of the xenograft tissue. Typically after surface carbohydrate moieties have been removed from living cells and the extracellular components, the living cells reexpress the surface carbohydrate moieties. Reexpression of antigenic moieties of a xenograft can provoke continued immunogenic rejection of the xenograft. In contrast, dead cells are unable to reexpress surface carbohydrate moieties. Removal of antigenic surface carbohydrate moieties from dead cells and the extracellular components of a xenograft substantially permanently eliminates antigenic surface carbohydrate moieties as a source of immunogenic rejection of the xenograft.

Accordingly, in the above-identified embodiments, the xenograft of the invention is subjected to freeze/thaw cycling as discussed above to disrupt, *i.e*., to kill the cells of the xenograft tissue. Once killed, the cells are no longer able to reexpress antigenic surface carbohydrate moieties such α-gal epitopes which are factors in the immunogenic rejection of the transplanted xenografts.

Either before or after the cells are killed, in embodiments of the invention, the xenograft is subjected to *in vitro* digestion of the xenograft with glycosidases, and specifically galactosidases, such as α-galactosidase, to enzymatically eliminate antigenic surface carbohydrate moieties. In particular, α-gal epitopes are eliminated by enzymatic treatment with α-galactosidases, as shown in the following reaction:

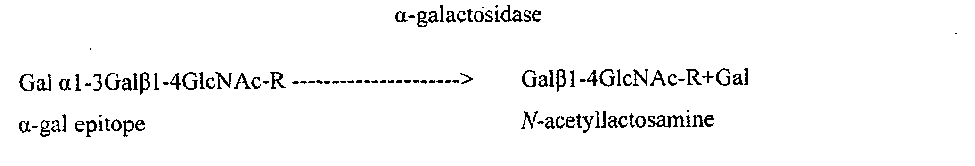

The *N*-acetyllactosamine residues are epitopes that are normally expressed on human and mammalian cells and thus are not immunogenic. The *in vitro* digestion of the xenograft with glycosidases is accomplished by various methods. For example, the xenograft can be soaked or incubated in a buffer solution containing glycosidase. In addition, the xenograft can be pierced to increase permeability, as further described below. Alternatively, a buffer solution containing the glycosidase can be forced under pressure into the xenograft via a pulsatile lavage process.

Elimination of the α-gal epitopes from the xenograft diminishes the immune response against the xenograft. The α-gal epitope is expressed in nonprimate mammals and in New World monkeys (monkeys of South America) as 1x10⁶-35x10⁶ epitopes per cell, as well as on macromolecules such as proteoglycans of the extracellular components. Galili U. et al., "Man, apes, and Old World monkeys differ from other mammals in the expression of α-galactosyl epitopes on nucleated cells." J. Biol. Chem. 263: 17755 (1988). This epitope is absent in Old World primates (monkeys of Asia and Africa and apes) and humans, however. *Id.* Anti-Gal is produced in humans and primates as a result of an immune response to α-gal epitope carbohydrate structures on gastrointestinal bacteria. Galili U. et al., "Interaction between human natural anti-α-galactosyl immunoglobulin G and bacteria of the human flora." Infect. Immun. 56: 1730 (1988); Hamadeh R.M. et al., "Human natural anti-Gal IgG regulates alternative complement pathway activation on bacterial surfaces" J. Clin. Invest. 89: 1223 (1992). Since nonprimate mammals produce α-gal epitopes, xenotransplantation of xenografts from these mammals into primates results in rejection because of primate anti-Gal binding to these epitopes on the xenograft. The binding results in the destruction of the xenograft by complement fixation and by antibody dependent cell cytotoxicity. Galili U et al., "Interaction of the natural anti-Gal antibody with α-galactosyl epitopes: A major obstacle for xenotransplantation in humans." Immunology Today 14: 480 (1993); Sandrin M et al., "Anti-pig IgM antibodies in human serum react predominantly with Galα1-3Gal epitopes." Proc. Natl. Acad. Sci. USA 90: 11391 (1993); GoodH et al., "Identification of carbohydrate structures which bind human anti-porcine antibodies: implications for discordant grafting in man." Transplant. Proc. 24: 559 (1992); Collins BH et al., "Cardiac xenografts between primate species provide evidence for the importance of the α-galactosyl determinant in hyperacute rejection." J. Immunol. 154: 5500 (1995). Furthermore, xenotransplantation results in major activation of the immune system to produce increased amounts of high affinity anti-Gal. In accordance with the invention, the substantial elimination of α-gal epitopes from cells and from extracellular components of the xenograft, and the prevention of reexpression of cellular α-gal epitopes diminish the immune response against the xenograft associated with anti-Gal antibody binding with α-gal epitopes.

In addition, the xenografts of the invention may be treated with polyethylene glycol (PEG) prior to or concurrently with treatment with glycosidase. PEG acts as a carrier for the glycosidase by covalently bonding to the enzyme and to the collagen extracellular components. Further, PEG-treated xenografts reduce immunogenicity.

Either before or after the xenograft cells are killed, in embodiments of the invention, the xenograft is washed or digested with one or more different types of proteoglycan-depleting factors. The proteoglycan-depleting factor treatment can precede or follow glycosidase treatment. Proteoglycans such as glycosaminoglycans (GAGs) are interspersed either uniformly as individual molecules or within varying amounts within the extracellular components of the invention's xenograft. The GAGs include mucopolysaccharide molecules such as chondroitin 4-sulfate, chondroitin 6-sulfate, keratan sulfate, dermatan sulfate, heparin sulfate, hyaluronic acid, and mixtures thereof. The proteoglycans including such GAGs contain attached carbohydrates such as α-gal epitopes. Such epitopes stimulate an immune response once the xenograft is transplanted, as discussed above. Washing or digesting the xenograft with the proteoglycan-depleting factor removes at least a portion of the proteoglycans and attached α-gal epitopes from the extracellular components of the xenograft, and thereby diminishes the immune response against the xenograft upon its transplantation. After the proteoglycan-depleting factor treatment and subsequent transplantation, natural tissue repopulates the remaining collagen shell.

Non-limiting examples of the proteoglycan-depleting factors used in the invention include proteoglycan-depleting factors such as chondroitinase ABC, hyaluronidase, chondroitin AC II lyase, keratanase, trypsin, fibrinectin and fragments of fibronectin.

Other proteoglycan-depleting factors known to those of ordinary skill in the art are also possible for use with the invention, however. The invention's xenograft is treated with proteoglycan-depleting factor in an amount effective for removing at least a portion of the proteoglycans from the extracellular components of the xenograft. Preferably, the xenograft is treated with proteoglycan-depleting factor such as hyaluronidase in an amount ranging from about 1.0 TRU/ml to about 100.0 TRU/ml or proteoglycan-depleting factor such as chondroitinase ABC in an amount ranging from about 0.01 u/ml to about 2.0 u/ml or most preferably, in an amount ranging from about 1.0 ul/ml to about 2.0 u/ml. The xenograft can also be treated with proteoglycan-depleting factor such as fibronectin fragment, (e.g., amino terminal 29-kDa fibronectin fragment) in an amount ranging from about .01 µM to about 1.0 µM, and preferably in an amount ranging from about 0.1 µM to about 1.0 µM.

During enzymatic and chemical processing, the head-space of the xenograft process solutions may be subjected to vacuum, either continuous or pulsed, to remove trapped air in the xenograft. For example, during enzymatic process incubation, the head-space above process solutions are exposed to vacuum of about 10 to 1000 millitorr and preferably 50 to 500 millitorr. The resulting influx of solution through the xenograft enhances permeation of the enzymatic or chemical agents.

Prior to treatment, the xenograft optionally may be pierced to increase permeability to agents used to render the xenograft substantially non-immunogenic. A sterile surgical needle such as an 18-gauge needle is used to perform this piercing step, or, alternatively a comb-like apparatus containing a plurality of needles are used. The piercing may be performed with various patterns, and with various pierce-to-pierce spacings, in order to establish a desired access to the interior of the xenograft. Piercing may also be performed with a laser. In one form of the invention, one or more straight lines of punctures about three millimeters apart are established circumferentially in the surface of the xenograft.

Prior to implantation, the xenograft of the invention may be treated with limited digestion by proteolytic enzymes such as ficin or trypsin to increase tissue flexibility, or coated with anticalcification agents, antithrombotic coatings, antibiotics, growth factors, or other drugs that may enhance the incorporation of the xenograft into the recipient. The xenograft of the invention may be further sterilized using known methods, for example, with additional glutaraldehyde or formaldehyde treatment, ethylene oxide sterilization, propylene oxide sterilization, or the like. The xenograft may be stored frozen until required for use.

The xenograft of the invention, or a segment thereof, may be implanted into a human by those of skill in the art using known surgical techniques, for example, by open-heart surgery, or minimally invasive techniques such as endoscopic surgery, and transluminal implantation. Specific instruments for performing such surgical techniques are known to those of skill in the art, which ensure accurate and reproducible placement of xenograft tissue.

*Tissue Sterilization:* The Food & Drug Administration (FDA) Center for Biologics Evaluation and Research (CBER) currently regulates human tissue intended for transplantation that is recovered, processed, stored, or distributed by methods that do not change tissue function or characteristics and that is not currently regulated as a human drug, biological product, or medical device. Examples of such tissues are bone, skin, corneas, ligament and tendon. The FDA (CBER) also regulates xenotransplantation, which is any procedure that involves the transplantation, implantation, or infusion into a human recipient of either (a) live cells, tissues, or organs from a nonhuman animal source or (b.) human body fluids, cells, tissues or organs that have had *ex vivo* contact with live nonhuman animal cells, tissues, or organs. Accordingly, tissue banks are required to have written procedures for prevention of infectious disease contamination or cross-contamination by tissue during processing.

Recently, the Minnesota Department of Health (MDH), in collaboration with United States Centers for Disease Control (CDC), has conducted an investigation of a young man who had died unexpectedly following knee surgery. CDC, " Public Health Dispatch: Update: Unexplained Deaths Following Knee Surgery --- Minnesota, 2001" MMWR 50(48); 1080 (December 7, 2001). The patient had received a knee osteochondral allograft. The tissue processor of the allograft had used aseptic processing of harvested tissues. Companion tissue had been processed alongside the allograft. After suspension of the allograft and companion tissue in an antibiotic/antifungal solution, the companion tissue was cultured. The aerobic and anaerobic cultures of the companion tissues were reported as negative. Nevertheless, blood cultures obtained from the patient before his death grew up chlostridial bacteria.

Because infection associated with contaminated graft tissue is a known complication of allograft surgery, the MDH, the CDC, and the FDA investigated whether the allograft might have been the source for the bacterial infection. Officials found twenty-five more cases of serious bacterial infections in people who received such operations. CDC, " Update: Allograft-Associated Bacterial Infections --- United States, 2002." MMWR 51(10); 207-210 (March 15, 2002); The New York Times, Online Edition (March 15, 2002).

Thirteen of the twenty-six patients were infected with chlostridial bacteria; eleven of these patients received tissue processed by the same tissue processor. Allografts that were implicated in *the* chlostridial infections were tendons used for anterior cruciate ligament (ACL) reconstruction, femoral condyles, bone, and meniscus. Eleven of the allografts were frozen and two were fresh (femoral condyles). All the allografts were processed aseptically but did not undergo terminal sterilization. MMWR 51(10); 207-210 (March 15, 2002).

Eleven patients were infected with gram-negative bacilli; five had polymicrobial infection. The transplanted tissues included ACL, femoral condyle, meniscus, and bone. One tissue was fresh (femoral condyle), one was freeze-dried (bone), and the rest were frozen. For eight of these thirteen cases, additional evidence implicated the allograft (*e*.*g*., common donors or positive pre-implantation or processing cultures with matching microorganisms). Eight patients received allografts that had undergone aseptic processing but no terminal sterilization. Three patients received allografts that were reported to have undergone gamma irradiation. MMWR 5(10); 207-210 (March 15, 2002).

In response, the CDC suggested some additional steps to reduce the risk for allograft-associated infections. "When possible, a method that can kill bacterial spores should be used to process tissue. Existing sterilization technologies used for tissue allografts, such as gamma irradiation, or new technologies effective against bacterial spores should be considered." MMWR 51(10); 207-210 (March 15,2002). Also, the FDA has released new guidelines for tissue processors regarding the processing of human tissues intended for transplantation. CBER, Guidance for Industry: Validation of Procedures for Processing of Human Tissues Intended for Transplantation (March 8, 2002).

The sterilization process of the invention provides bioburden and viral inactivation by a combination of two processing steps from the tissue treatment process described above.

The first step is a chemical sterilization treatment with glutaraldehyde, and the second step is terminal sterilization by electron beam irradiation. The chemical sterilization step involves tissue incubation in 0.10% glutaraldehyde for 9 to 16 hours at 20°C to 25°C. Tissue glutaraldehyde penetration was validated by hydrothermal shrink temperature assay.

The second step is a terminal sterilization based on ANSI/AAMI/ISO 11137 medical device sterility assurance limits, and uses electron beam irradiation as a controlled ionizing radiation source. Medical device sterility assurance is based on validation of a sterilization process that can repeatedly produce medical devices with a sterility assurance limit that meets the current standards (SAL = 10⁻⁶).

In one embodiment of the tissue sterilization process of the invention, the Z-Lig device (a device of the invention) is terminally sterilized using E-beam ionizing radiation. For this embodiment, the validated sterilization dose of 17.8 kGy was established using ANSI/AAMI/ISO 11137-Dose Method 1 to provide a sterility assurance level of 10⁻⁶. Further, a range of sterilization dose from 15.8 to 21.3 kGy has utility as a terminal sterilization dose.

To assure that all devices within a single processing load receive the minimum dose, as established by the validation study, a dose mapping study was conducted on a standard packaging configuration of the device. See, EXAMPLE 1. The EXAMPLE verifies that all devices within the standard packaging would receive the validated dose of 17.8 kGy of ionizing radiation.

Together the embodiment and EXAMPLE 1 validate the capability of this manufacturing process to reproducibly meet the requirements established in ANSI/AAMI/ISO 11137.

### EXAMPLE 1

### SOURCE MATERIAL CONTROL AND VIRAL INACTIVATION

The viral safety of the porcine device was evaluated by assessment of the animal source profile and evaluation of the viricidal activity of the treatment process. The tissues used originate from six-month-old animals from a closed swine herd. Animals are subjected to an ante and post mortem health inspection by licensed veterinarians and processed in a USDA-inspected facility. Tissue identification allows tracking of harvested materials forward to the finished product and back-wards to the animal of origin. No modified live viral vaccines are used for disease control in the production facility. There is no cross contamination with other animal sourced materials at any point in the harvesting or manufacturing processes. Viral reduction values of greater than six-logs were observed for *porcine parvovirus, influenza A, pseusdorabies virus* and *reovirus* 3 during an evaluation of the viricidal activity of two steps within the treatment process (glutaraldehyde treatment and electron beam irradiation, 17.8 kGys). In other words, the resulting xenograft material was "substantially free" of these three viruses.

Results from both glutaraldehyde and electron beam irradiation viral inactivation steps are shown in TABLE 1 below.

**TABLE 1**

| Viral Inactivation Test Results | | | | |
|---|---|---|---|---|
| Virus | Description | Log₁₀ Reduction Glutaraldehyde | Log₁₀ Reduction E-beam | Total Process Reduction (Log₁₀) |
| Influenza A | Env./RNA | NT | 6.52 | 6.52 |
| Porcine | NEnv./DN | 1.91 | 5.06 | 6.97 |
| Parvovirus | A | | | |
| Pseudorabies | Env./DNA | NT | 6.30 | 6.30 |
| Reovirus 3 | NEnv./RN | 4.24 | 7.47 | 11.71 |
| | A | | | |

| | | | | |
|---|---|---|---|---|
| Env = enveloped, NEnv = non-enveloped, NT = Not Tested | | | | |

*Porcine Endogeneous Retrovirus*: Porcine endogenous retrovirus (PERV) has been discussed as a potential risk from xenogeneic materials. Takefman DM, Wong S. Maudru et al. "Detection and characterization of porcine endogeneous retrovirus in porcine plasma and porcine factor VII. "J. Virol 75(10):4551 (2001). We evaluated the risk of PERV based on three separate assays.

The first assay was a standard viral inactivation assay of non-endogenous murine leukemia virus as a model virus for PERV. The model virus inactivation study was conducted under similar test conditions to the viral inactivation studies in the previous section. The log reduction value was calculated to be 4.21 by these test methods.

In the second assay, finished Z-Lig device material (an embodiment of the invention) was submitted for a co-cultivation assay for PERV. Samples were co-cultivated with human U293 cells to assess transfection of endogenous PERV from the ligament to the human cell line. The assays were conducted according to previously published methods. Takefman DM, Wong S. Maudru et al. "Detection and characterization of porcine endogeneous retrovirus in porcine plasma and porcine factor VII." J. Virol 75(10):4551 (2001). Non-transfection of PERV was measurable by the methods utilized.

The third assay evaluated device cellular inactivation by the following three manufacturing steps: (1) freeze/thaw (a minimum of two freeze/thaw cycles); (2) incubation with 0.10% glutaraldehyde and (3) exposure to 17.8 kGy ionizing radiation. The cytotoxicity potential of these steps has an estimated safety margin of 108.9 for cell kill.

To verify the lack of live cells in the device, we performed a ³⁵S labeled methionine uptake study. The cellular uptake of radiolabeled methionine is measured in a scintillation counter. Live cells incorporate this labeled amino acid as part of their normal metabolism. No methionine uptake was observed in suspensions of device fragments incubated under cell culture conditions for 48 hours. No metabolic activity, consistent with cell viability within the Z-Lig device was observed.

Based on the results of these assays and the apparent lack of viable cells in the treated device, it was concluded that the risk of PERV transmission from this device to a human recipient is extremely low.

### EXAMPLE 2

### TRANSMISSIBLE SPONGIFORM ENCEPHALOPATHY

The transmissible spongiform encephalopathies (TSE) family of diseases includes scrapie, which affects sheep and goats; transmissible mink encephalopathy; feline spongiform encephalopathy; chronic wasting disease of deer and elk; and in humans, kuru, both classic and variant Creutzfeldt-Jakob disease, Gerstmann-Straussler-Scheinker syndrome, and fatal familial insomnia. Bovine spongiform encephalopathy (BSE), widely referred to as "mad cow disease," is a chronic degenerative disease affecting the central nervous system of cattle. TSE's have also been reported in captive exotic ruminants, and exotic and domestic cats. The agent isolated from several of these cases is indistinguishable from BSE in cattle suggesting the occurrence of TSE's in these species resulted from BSE-contaminated feed.

The nature of the infectious agent that causes BSE and scrapie is unknown. Currently, the most accepted theory is that the agent is a modified form of a normal cell protein known as a prion.

Tissues for the Z-Lig device (an embodiment of the invention) are sourced from a closed swine herd within the United States. The animals are managed under an intensive herd health and disease-monitoring program. The slaughter facility where the tissues are harvested is USDA inspected and individual animal to device trace-ability exists. Based on the current understanding of the absence of TSE in cattle and swine in the United States and the exclusion of species known to have TSE in the United States from the feed supply of the source animals, it can be concluded that the risk of transmission of TSE from the Z-Lig to human recipients is extremely low and consistent with the risk associated with other porcine tissue based devices currently approved for sale in the US by the FDA. In other words, the resulting xenograft material is "substantially free" of these a TSE agents.

### EXAMPLE 3

### BIOMECHANICAL TESTING

As part of process development, we initiated tests to characterize the pre-implantation biomechanical properties of bone-patellar tendon-bone allografts. We have implemented clinically relevant controls for comparative biomechanical evaluation. Anatomical, structural and cellular similarities between pig and human patellar tendon have been documented and support porcine patellar tendon as a viable choice for human graft biomechanical modeling and alternative. Fuss FK. "Anatomy and function of the cruciate ligaments of the domestic pig (Sus scrofa donaestica): a comparison with human cruciates." J Anat 178: 11 (October 1991). The overall aim of the testing was an internally controlled comparison of the Z-Lig anterior cruciate ligament replacement device (an embodiment of the invention) to human bone-patellar tendon-bone constructs. An additional control group included unprocessed porcine patellar tendon, treated and harvested as cadaveric grafts fresh frozen with no additional treatments. Test groups and descriptions are presented in TABLE 2.

**TABLE 2**

| Biomechanical Test Groups | | |
|---|---|---|
| Group | Abbreviation | Description |
| 1 | Z-Lig (pPT-treated) | Porcine bone-patellar tendon-bone treated with oc-galactosidase, 0.10% glutaraldehyde, 17.8 kGy e-beam |
| 3 | pPT-untreated | Porcine bone-patellar tendon-bone, fresh frozen, untreated |
| 3 | hPT | Human bone-patellar tendon-bone, fresh frozen |

Previously published results have evaluated the tensile biomechanical and structural properties of human anterior cruciate ligament, gracilis tendon, semitendinosus and patellar tendon. Gibbons ML et.al. "Effects of gamma irradiation on the initial mechanical and material properties of goat bone-patellar tendon-bone allografts." J Orthop Res 9(2): 209 (March 1991); Smith CW et al.. "Mechanical properties of tendons: changes with sterilization and preservation." J Biomech Eng 118(1): 56-61 (February 1996).

The aim of this EXAMPLE was to compare both structural and material properties of treated and untreated porcine patellar tendon grafts and a comparison to properties of human patellar tendon. The results of this EXAMPLE serve as internally controlled comparative evaluations and validations of test methods.

*Materials and Methods:* Biomechanical evaluation incorporates design and test numbers recommended by the FDA, Guidance Document for the Preparation of Investigational Device Exemptions and Premarket Approval Applications For Intra-Articular Prosthetic Knee Ligament Devices (February 18, 1993). Three test groups with a minimum of eight specimens per group are used in this study. The test groups include the Z-Lig device, human patellar tendon allograft and untreated porcine patellar tendon grafts. All testing used fresh-frozen grafts stored frozen, then thawed just before testing.

Both bone-to-bone length and mid-substance cross-sectional area were measured for tested ligaments. The mid-substance thickness was measured with uniform load (0.12MPA) and accomplished by a standard 10 mm blocking channel, 500g weight with 40 mm² load surface. Specially designed screw clamp fixtures were used to hold the test specimens at the bone plug ends, with compression in the anterior-posterior plane to 20 in lbs by set screws. The clamps were then vertically submerged in a cylindrical acrylic chamber containing 37°C saline. The upper clamp was mounted to the actuator of a servo-control hydraulic test machine (Shore Western Materials Testing Systems). The lower clamp was fixed to the bottom of the chamber. Mechanical loading was produced with a servo-controlled hydraulic test machine and measured by a 1000 1b load cell. (Shore Western Materials Testing Systems). An EnduraTEC WinTest™ Control system was used to operate the test frame, with digital data acquisition at 200Hz and a standard strain rate of 100%/sec (based on bone-to-bone length).

The collected data were plotted in both load vs. displacement plots and normalized into stress vs. strain plots. The following structural properties were determined from load displacement curves: ultimate load, ultimate displacement, yield load, yield displacement, axial stiffness, and toe region. Axial stiffness was calculated from best-fit linear analysis using the linear slope region of the load-displacement plot, with the toe region the initial non-linear region and yield point determined by upper proportional limit and deviation from linearity. Conversion of these tensile properties was accomplished by normalization of stress vs. strain plots and specimen cross-sectional area. Stress is equal to load divided by cross-sectional area and strain is derived by specimens elongation as compared to initial bone-to-bone length. Material properties reported for specimens and groups include: yield strength, ultimate strength, yield strain, ultimate strain, and modulus. Statistical significance for all parameters was first assessed by ANOVA, with post-hoc testing performed by t-test at a significance level of p<0.05.

*Results:* The physical characterization of the specimens is shown in TABLE 3 below. Only specimens tested to failure under valid test conditions were used for this analysis. Mean values in millimeters are reported for each test group with variance reported as standard deviation.

**TABLE 3**

| Length and Cross-sectional Area of Tensile Biomechanical Test Groups | | | |
|---|---|---|---|
| | Z-Li (n=8) | pPT-untreated (n=8) | hPT (n=13) |
| BTB Length (mm) (mean) | 48.0 ± 3.2 | 56.2 ± 3.4 | 43.2 ± 5.6 |
| Cross-sectional area (mm²) (mean) | 60.3 ± 10.7 | 57.8 ± 13.2 | 36.6 ± 4.9 |

Length of the Z-Lig and pPT groups, 48.0 and 56.2 mm respectively, were significantly greater than hPT length (43.2 mm, p<0.05) with non-significant differences between the porcine test groups. The porcine test groups were significantly greater in cross-sectional area as compared to hPT (p≤ 0.05).

The structural properties of ultimate load, yield load, ultimate displacement, yield displacement, and stiffness are presented in TABLE 4 below. Although differences are seen, they are attributed to disparate cross-sectional areas between the groups. Normalized inter-group analysis is accomplished by structural to material property conversion and comparison as shown in TABLE 5.

**TABLE4**

| Structural Properties of Tensile Biomechanical Test Groups | | | |
|---|---|---|---|
| | Z-Lig (n=8) | pPT-untreated (n=8) | hPT (n=13) |
| Ultimate Load (N) (mean) | 1793 ± 356 | 1847 ± 472 | 1139 ± 214 |
| Yield Load (N) (mean) | 1525 ± 386 | 1424 ± 505 | 954 ± 210 |
| Ultimate Displacement (mm) (mean) | 16.3 ± 1.7 | 17.0 ± 2.4 | 12.1 ± 2.3 |
| Yield Displacement (mm) (mean) | 13.6 ± 1.32 | 12.3 ± 1.42 | 9.9 ± 2.3 |
| Stiffness (N/mm) (mean) | 189 ± 31 | 184 ± 41 | 180 ± 42 |

**TABLE 5**

| Material Properties of Tensile Biomechanical Test Groups | | | |
|---|---|---|---|
| | Z-Lig (n=8) | pPT-untreated (n=8) | hPT (n=13) |
| Ultimate Strength (MPa) (mean) | 30.2 + 6.9 | 32.7 + 8.2 | 31.77 + 8.4 |
| Yield Strength (MPa) (mean) | 25.6 + 7.1 | 25.1 + 7.0 | 26.4 + 7.1 |
| Ultimate Strain (%) (mean) | 34.1 + 4.2% | 31.5 + 3.2% | 28.2 + 4.8% |
| Yield Strain (%) (mean) | 28.4 + 3.3% | 22.1 + 2.1% | 23.1 + 5.2% |
| Modulus (MPa) (mean) | 151.3 + 18.5 | 184.0 + 47.9 | 217.9+ 78.0 |

The details of one or more embodiments of the invention are set forth in the accompanying description above. Although any methods and materials similar or equivalent to those described herein can be used in the practice or testing of the invention, the preferred methods and materials are now described. Those of skill in the art will recognize that the invention may be embodied in other specific forms without departing from the essential characteristics thereof. Other features, objects, and advantages of the invention will be apparent from the description and from the claims. In the specification and the appended claims, the singular forms include plural referents unless the context clearly dictates otherwise. Unless defined otherwise, all technical and scientific terms used herein have the same meaning as commonly understood by one of ordinary skill in the art to which this invention belongs.

The foregoing description has been presented only for the purposes of illustration and is not intended to limit the invention to the precise form disclosed, but by the claims appended hereto.

## Claims

1. A method of sterilizing xenograft cartilaginous structures, ligaments, tendons and heart valves for implantation in a human, comprising the steps of:
(1) obtaining substantially non-immunogenic xenograft material;
(2) treating the xenograft material with at least one crosslinking agent; and
(3) subjecting the crosslinked xenograft material to a dose of sterilizing electron-beam radiation treatment of between 15.8 to 21.3 kGy.

2. The method of claim 1, wherein the xenograft material is substantially depleted in carbohydrate chains having terminal α-galactosyl sugar at the non-reducing end of the carbohydrate chain.

3. The method of claim 1, wherein the substantially non-immunogenic xenograft is substantially depleted in a proteoglycans.

4. The method of claim 1, wherein the substantially non-immunogenic xenograft material is soft tissue.

5. The method of claim 1, wherein the substantially non-immunogenic xenograft material is heart valve tissue.

6. The method of claim 1, wherein the substantially non-immunogenic xenograft material is porcine.

7. The method of claim 1, wherein the substantially non-immunogenic xenograft material is sterilized.

8. The method of claim 7, wherein the sterilizing is by with one or more agents selected from the group consisting of ethylene oxide, and propylene oxide.

9. The method of claim 1, wherein the crosslinking agent is selected from the group consisting of aldehydes, aromatic diamines, carbodiimiides, and diisocyanates.

10. The method of claim 1, wherein at least one crosslinking agent is glutaraldehyde.

11. The method of claim 1, wherein the crosslinking agent is a solution containing about 0.01 percent to about 5 percent glutaraldehyde.

12. The method of claim 1, wherein the crosslinking treatment is by exposing the xenograft material to a crosslinking agent in a vapor form.

13. The method of claim 1, wherein the radiation treatment comprises a validated sterilization dose of the 17.8 kGy.

14. The method of claim 1, wherein the sterilized xenograft material has a sterility assurance level of at least 10⁻⁶.

15. The method of claim 1, wherein the sterilized xenograft material is substantially free of a virus selected from the group consisting of porcine parvovirus, influenze A, pseudorabies virus and reovirus 3.

16. The method of claim 1, wherein the sterilized xenograft material is substantially free of a transmissible spongiform encephalopathy (TSE) agent.

17. The method of claim 1, wherein the sterilized xenograft material has substantially the same mechanical properties as the native heart valve.

## Patentansprüche

1. Verfahren zum Sterilisieren von Knorpelstruktur-, Ligament-, Sehnen- und Herzklappen-Xenotransplantaten für die Implantation in einen Menschen, umfassend die Schritte:
(1) Erhalten von im Wesentlichen nicht-immunogenem Xenotransplantatmaterial,
(2) Behandeln des Xenotransplantatmaterials mit mindestens einem Vernetzungsmittel, und
(3) Aussetzen des vernetzten Xenotransplantatmaterials einer Behandlung mit einer Dosis sterilisierender Elektronenstrahl-Strahlung von zwischen 15,8 bis 21,3 kGy.

2. Verfahren nach Anspruch 1, wobei das Xenotransplantatmaterial im Wesentlichen an Kohlehydratketten, die einen terminalen α-Galactosyl-Zucker am nichtreduzierenden Ende der Kohlehydratkette aufweisen, abgereichert ist.

3. Verfahren nach Anspruch 1, wobei das im Wesentlichen nicht-immunogene Xenotransplantat im Wesentlichen an Proteoglycanen abgereichert ist.

4. Verfahren nach Anspruch 1, wobei das im Wesentlichen nicht-immunogene Xenotransplantatmaterial weiches Gewebe ist.

5. Verfahren nach Anspruch 1, wobei das im Wesentlichen nicht-immunogene Xenotransplantatmaterial Herzklappengewebe ist.

6. Verfahren nach Anspruch 1, wobei das im Wesentlichen nicht-immunogene Xenotransplantatmaterial vom Schwein stammt.

7. Verfahren nach Anspruch 1, wobei das im Wesentlichen nicht-immunogene Xenotransplantatmaterial sterilisiert ist.

8. Verfahren nach Anspruch 7, wobei das Sterilisieren mit einem oder mehreren Mitteln, ausgewählt aus der Gruppe, bestehend aus Ethylenoxid und Propylenoxid, erfolgt.

9. Verfahren nach Anspruch 1, wobei das Vernetzungsmittel aus der Gruppe, bestehend aus Aldehyden, aromatischen Diaminen, Carbodiimide und Diisocyanaten, ausgewählt ist.

10. Verfahren nach Anspruch 1, wobei mindestens ein Vernetzungsmittel Glutaraldehyd ist.

11. Verfahren nach Anspruch 1, wobei das Vernetzungsmittel eine Lösung ist, enthaltend etwa 0,01% bis etwa 5% Glutaraldehyd.

12. Verfahren nach Anspruch 1, wobei die Vernetzungsbehandlung durch Aussetzen des Xenotransplantatmaterials einem Vernetzungsmittel in Dampfform erfolgt.

13. Verfahren nach Anspruch 1, wobei die Strahlungsbehandlung eine bestätigte Sterilisierungsdosis von 17,8 kGy umfaßt.

14. Verfahren nach Anspruch 1, wobei das sterilisierte Xenotransplantatmaterial ein Sterilitätssicherheitsniveau von mindestens 10⁻⁶ aufweist.

15. Verfahren nach Anspruch 1, wobei das sterilisierte Xenotransplantatmaterial im Wesentlichen frei von einem Virus, ausgewählt aus der Gruppe, bestehend aus Schweineparvovirus, Influenza A, Pseudorabies-Virus und Reovirus 3, ist.

16. Verfahren nach Anspruch 1, wobei das sterilisierte Xenotransplantatmaterial im Wesentlichen frei von einem übertragbare spongiforme Enzephalopathie (*transmissible spongiform encephalopathy,* TSE)-Erreger ist.

17. Verfahren nach Anspruch 1, wobei das sterilisierte Xenotransplantatmaterial im Wesentlichen die gleichen mechanischen Eigenschaften wie die native Herzklappe aufweist.

## Revendications

1. Procédé de stérilisation de structures cartilagineuses de xénogreffe, tendons et valvules cardiaques à implanter dans un être humain, comprenant les étapes consistant à :
(1) obtenir un matériau de xénogreffe sensiblement non-immunogène ;
(2) traiter le matériau de xénogreffe avec au moins un agent réticulant ; et
(3) soumettre le matériau de xénogreffe réticulé à une dose de traitement par radiation à faisceau électronique de stérilisation entre 15,8 et 21,3 kGy.

2. Procédé selon la revendication 1, dans lequel le matériau de xénogreffe est sensiblement exempt de chaînes de carbohydrate ayant un sucre de α-galactosyle terminal à l'extrémité non-réduetrice de la chaîne de carbohydrate.

3. Procédé selon la revendication 1, dans lequel la xénogreffe sensiblement non-immunogène est sensiblement exempt d'un protéoglycane.

4. Procédé selon la revendication 1, dans lequel le matériau de xénogreffe sensiblement non-immunogène est un tissu mou.

5. Procédé selon la revendication 1, dans lequel le matériau de xénogreffe sensiblement non-immunogène est un tissu de valvule cardiaque.

6. Procédé selon la revendication 1, dans lequel le matériau de xénogreffe sensiblement non-immunogène est porcin.

7. Procédé selon la revendication 1, dans lequel le matériau de xénogreffe sensiblement non-immunogène est stérilisé.

8. Procédé selon la revendication 7, dans lequel la stérilisation s'effectue par un ou plusieurs agents choisis dans le groupe constitué d'oxyde d'éthylène, et d'oxyde de propylène.

9. Procédé selon la revendication 1, dans lequel l'agent de réticulation est choisi dans le groupe constitué d'aldéhydes, de diamines aromatiques, de carbodiimides et de diisocyanates.

10. Procédé selon la revendication 1, dans lequel au moins un agent de réticulation est un glutaraldéhyde.

11. Procédé selon la revendication 1, dans lequel l'agent de réticulation est une solution contenant environ 0,01 pour cent à environ 5 pour cent de glutaraldéhyde.

12. Procédé selon la revendication 1, dans lequel le traitement de réticulation s'effectue en exposant le matériau de xénogreffe à un agent de réticulation sous forme de vapeur.

13. Procédé selon la revendication 1, dans lequel le traitement de radiation comprend une dose de stérilisation validée de 17,8 kGy.

14. Procédé selon la revendication 1, dans lequel le matériau de xénogreffe stérilisé a un niveau d'assurance de stérilité d'au moins 10⁻⁶.

15. Procédé selon la revendication 1, dans lequel le matériau de xénogreffe stérilisé est sensiblement dénué d'un virus, choisi dans le groupe constitué du parvovirus porcin, du virus influenza A, du virus de la pseudorage et de réovirus 3.

16. Procédé selon la revendication 1, dans lequel le matériau de xénogreffe stérilisé est sensiblement dénué d'un agent de l'encéphalopathie spongiforme transmissible (TSE).

17. Procédé selon la revendication 1, dans lequel le matériau de xénogreffe stérilisé a sensiblement les mêmes propriétés mécaniques que la valvule cardiaque native.
